# EUROPEAN PATENT SPECIFICATION

(11) **EP 1 806 120 B1**
(45) Date of publication and mention of the grant of the patent: **22.05.2013**
(21) Application number: 05785273.3
(22) Date of filing: 21.09.2005
(51) Int. Cl.: A61Q 19/02, A61K 8/35, A61K 8/60, A61K 8/97, A61K 31/05

(54) **COMPOSITION FOR PREVENTION OR ALLEVIATION OF PIGMENTATION**
ZUSAMMENSETZUNG ZUR PRÄVENTION ODER LINDERUNG VON PIGMENTIERUNG
COMPOSITION POUR PREVENIR OU DIMINUER LA PIGMENTATION

(30) Priority: 22.09.2004 JP 2004274454; 27.12.2004 JP 2004376562; 01.07.2005 JP 2005194428
(43) Date of publication of application: 11.07.2007
(73) Proprietor: Otsuka Pharmaceutical Co., Ltd., Tokyo 101-8535 (JP)
(72) Inventor: HARANO, Fumiki, c/o OTSUKA PHARMACEUTICAL CO., LTD, Otsu-shi, Shiga 520-0002 (JP); SHINOHARA, Shigeo c/o OTSUKA PHARMACEUTICAL CO., LTD, Otsu-shi, Shiga 520-0002 (JP); TANAKA, Masahiko c/o OTSUKA PHARMACEUTICAL CO., LTD., Otsu-shi, Shiga 520-0002 (JP)
(74) Representative: HOFFMANN EITLE
(86) International application number: PCT/JP2005/017363
(87) International publication number: WO 2006/033343

(56) References cited:
- EP-A- 0 904 774
- EP-A- 1 455 744
- WO-A-03/032941
- WO-A-03/084485
- WO-A-03/097072
- WO-A-2004/016238
- WO-A1-02/11717
- FR-A- 2 787 710
- JP-A- 6 048 934
- JP-A- 10 007 541
- JP-A- 2002 284 664
- JP-A- 2004 075 645
- JP-A- 2004 238 386
- US-B1- 6 300 369

## Description

### TECHNICAL FIELD

The present invention relates to a composition for preventing or improving pigmentation of the skin that can prevent or improve effectively skin pigmentation. The present invention also relates to a non-therapeutic method to this end.

### BACKGROUND OF THE INVENTION

Pigmentation of the skin, such as chloasma, freckles, and the like, is a serious aesthetic problem particularly for women. Generally, the onset of chloasma and freckles on the skin is induced by the precipitation of melanin pigment formed in the skin cells when the skin is stimulated by exposure to ultraviolet light and the like. Therefore, pigmentation of the skin, such as chloasma, freckles, and the like, has heretofore been prevented or improved by the use of an externally-applied composition comprising arbutin, kojic acid, ascorbic acid, ascorbic acid derivatives, ellagic acid, 4-n-butylresorcinol, linolic acid, tranexamic acid, chamomile extract, etc., which directly or indirectly inhibit the formation of melanin pigment (e.g., Non-patent Document 1). However, these externally-applied compositions are disadvantageous in that the effect of improving the pigmentation is slow and insufficient. Ubiquinone is known to have an antioxidant action, but the effect of preventing or improving pigmentation is inadequate and is not satisfactory.

In contrast, Adenosine 5'-monophosphate (hereinafter referred to as AMP) is already known to have an action for improving the pigmentation of the skin, and can be suitably used in externally-applied compositions for improving the pigmentation of the skin. WO 2004/016238 describes a composition comprising Adenosine-5'-monophosphate and ascorbic acid.

However, no case to date has been reported in which a specific melanin formation inhibitor or ubiquinone as defined in claim 1 is used in combination with AMP until now, and thus the effect obtained by the combined use thereof is not known.

### [Non-patent Document 1]

"Keshohin no Yuyosei: Hyokagijutu no Shinpo to Shorai Tenbo" (Functional Cosmetology Substantiation of Cosmetics Efficacy: Recent Progress and Future Promise of Evaluation Techniques); edited by Katsuiyuki TAKEDA, Shotaro HARADA, and Masanori ANDO; Nippon Keshohin Gijutsushakai;Yakuji Nippo; pp. 149 to 159.

### DISCLOSURE OF THE INVENTION

### PROBLEM TO BE SOLVED BY THE INVENTION

The present invention aims to provide a composition for preventing or improving pigmentation of the skin that can effectively prevent or improve the skin pigmentation. The present invention also aims to provide a non-therapeutic method for the purposes defined in claim 5.

### MEANS FOR SOLVING THE PROBLEM

The present inventors carried out extensive research to solve the above-described problems, and found that the action of preventing or improving pigmentation of the skin is synergistically enhanced by the combined use of at least one member (A) selected from the group consisting of AMP and salts thereof; and at least one member (B) selected from the group consisting of arbutins, ellagic acid, 4-alkylresorcinols, tranexamic acid, salts thereof, chamomile extracts, and ubiquinones. The inventors conducted further research and accomplished the present invention based on these findings.

The present invention is defined in claims 1-5.

### BRIEF DESCRIPTION OF THE DRAWINGS

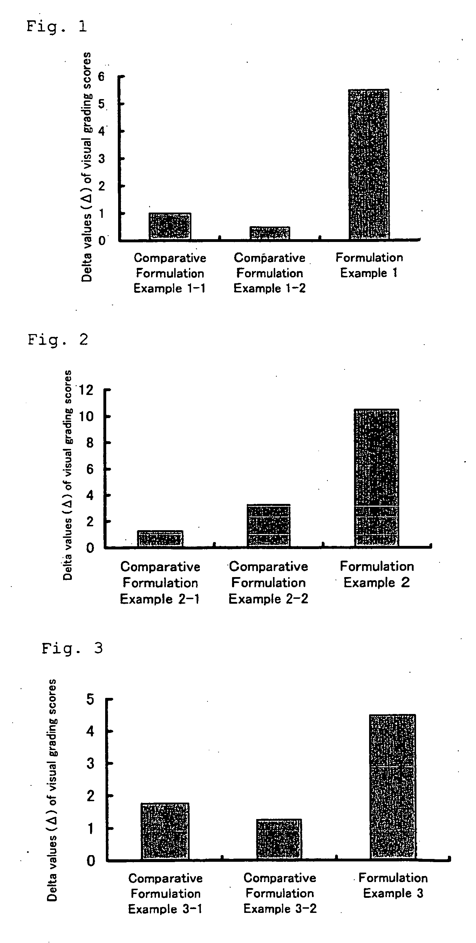

Fig. 1 shows delta values (A) of visual grading scores (indexes showing the improvement effects of skin pigmentation) of Formulation Example 1 (combined use of AMP2Na and arbutin), Comparative Formulation Example 1-1 (AMP2Na), and Comparative Formulation Example 1-2 (arbutin) in Test Example 1.
Fig. 2 shows delta values (Δ) of visual grading scores (indexes showing the improvement effects of skin pigmentation) of Formulation Example 2 (combined use of AMP2Na and ellagic acid hydrate), Comparative Formulation Example 2-1 (AMP2Na), and Comparative Formulation Example 2-2 (ellagic acid hydrate) in Test Example 2.
Fig. 3 shows delta values (Δ) of visual grading scores (indexes showing the improvement effects of skin pigmentation) of Formulation Example 3 (combined use of AMP2Na and tranexamic acid), Comparative Formulation Example 3-1 (AMP2Na) and Comparative Formulation Example 3-2 (tranexamic acid) in Test Example 3.

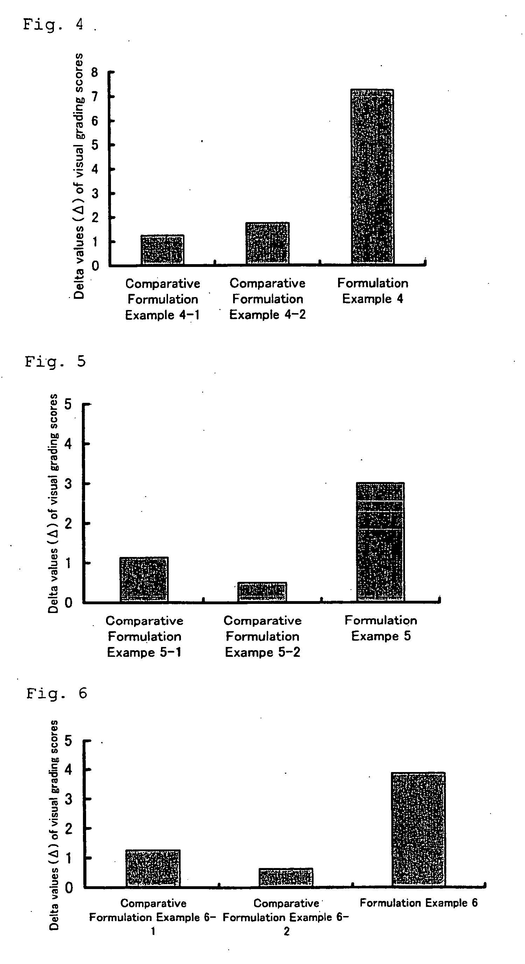

Fig. 4 shows delta values (A) of visual grading scores (indexes showing the improvement effects of skin pigmentation) of Formulation Example 4 (combined use of AMP2Na and 4-n-hexylresorcinol), Comparative Formulation Example 4-1 (AMP2Na) and Comparative Formulation Example 4-2 (4-n-hexylresorcinol) in Test Example 4.
Fig. 5 shows delta values (Δ) of visual grading scores (indexes showing the improvement effects of skin pigmentation) of Formulation Example 5 (combined use of AMP2Na and chamomile extract), Comparative Formulation Example 5-1 (AMP2Na) and Comparative Formulation Example 5-2 (chamomile extract) in Test Example 5.
Fig. 6 shows delta values (A) of visual grading scores (indexes showing the improvement effects of skin pigmentation) of Formulation Example 6 (combined use of AMP2Na and coenzyme Q10), Comparative Formulation Example 6-1 (AMP2Na) and Comparative Formulation Example 6-2 (coenzyme Q10) in Test Example 6.

### BEST MODE FOR CARRYING OUT THE INVENTION

Hereinafter, the present invention will be described in detail.

### 1. Composition for preventing or improving skin pigmentation

The composition for preventing or improving skin pigmentation of the invention comprises at least one member (hereinafter referred to as ingredient(s) (A)) selected from the group consisting of AMP and salts thereof.

In the invention, there is no limitation on the salts of AMP used as the ingredient (s) (A) insofar as they can be added to cosmetics and externally-applied pharmaceutical compositions. Specific examples of the salts of AMP include alkali metal salts such as sodium salts, potassium salts, and the like; alkaline earth metal salts such as calcium salts, magnesium salts, barium salts, and the like; basic amino acid salts, such as arginine, lysine, and the like; ammonium salts, such as ammonium salts, tricyclohexyl ammonium salts, and the like; various alkanolamine salts such as monoethanolamine salts, diethanolamine salts, triethanolamine salts, monoisopropanolamine salts, diisopropanolamine salts, and triisopropanolamine, and the like. Among these, alkali metal salts, such as sodium salts and the like, are preferable. Specific examples of such alkali metal salts include adenosine monophosphate monosodium and adenosine monophosphate disodium. These salts of AMP may be used singly or in combination.

The proportion of the ingredient(s) (A) added to the composition of the invention is not limited and can be suitably adjusted according to the form of the composition, kind of the ingredient(s) (A), expected effect, etc. As one example, the total proportion of the ingredient(s) (A) is in the range of 0.05 to 10% by weight, preferably 0.1 to 7% by weight, and more preferably 0.5 to 6% by weight based on the total weight of the composition for preventing or improving skin pigmentation.

The composition for preventing or improving skin pigmentation of the invention comprises at least one member (hereinafter referred to as ingredient(s) (B)) selected from the group consisting of arbutins (chemical name: 4-hydroxyphenyl-β-D-glucopyranoside and 4-Hydroxyphenyl-α-D-glucopyranoside), ellagic acid, 4-alkylresorcinols, tranexamic acid, salts thereof, chamomile extracts, and ubiquinones in addition to the ingredient(s) (A).

The above-mentioned arbutin may be either α- or β-arbutin.

Specific examples of the above-mentioned 4-alkylresorcinol include 4-methylresorcinol, 4-ethylresorcinol, 4-n-propylresorcinol, 4-n-butylresorcinol, 4-n-pentylresorcinol, 4-n-hexylresorcinol, etc. Among these, 4-n-butylresorcinol, 4-n-pentylresorcinol, and 4-n-hexylresorcinol are preferable, and 4-n-butylresorcinol and 4-n-hexylresorcinol are more preferable.

Among the above-mentioned ingredients (B), arbutin, ellagic acid, 4-alkylresorcinol, and tranexamic acid may be in the form of a salt insofar as they are cosmetically or pharmaceutically acceptable. Examples of these salts include alkali metal salts, alkaline earth metal salts, basic amino acid salts, ammonium salts, alkanolamine salts, etc. as well as above-mentioned examples of the salts of AMP.

Chamomile extracts are extracts obtained by extracting. flower and/or stem of Matricaria chamomilla using a solvent. Specifically, chamomile extracts can be obtained by extracting from the flower and/or stem of Matricaria chamomilla in water or an organic solvent, or a mixed liquid of water and an organic solvent at normal temperature or under warmed conditions. The flower and/or stem may be used as it is, or, as required, may be dried, chopped, crushed, compressed, or boiled for the extraction step. Examples of the organic solvent used for this extraction process include lower alcohols with a carbon number range of C1-C5 (e.g., methanol, ethanol, propanol, isopropanol, butanol, etc.), propylene glycol, 1,3-butylene glycol, acetone, ethyl acetate, chloroform, toluene, pentane, hexane, heptane, etc. and these solvents can be used singly or in combination. As the extracting solvent, water, lower alcohols with a carbon number range of C1-C5, or a mixture of such alcohols and water is preferable, and ethanol and the mixture of ethanol and water are particularly preferable. In the case of the mixture of lower alcohols with a carbon number range of C1-C5 and water, the proportion of the alcohol based on the total weight of the mixture is, for example, 0.01 to 100% by weight, preferably 5 to 55% by weight, and more preferably 45 to 55% by weight. Chamomile extracts are commercially available, and such commercially-available chamomile extracts can be used.

In the invention, chamomile extracts may be used in the form of an extracted liquid, an extracted concentrate, purified matter, dried matter, a solution made using dried matter, or fractioned matter, etc.

There is no limitation on the above-mentioned ubiquinones, and, for example, coenzyme Q6, coenzyme Q7, coenzyme Q8, coenzyme Q9, and coenzyme Q10 can be mentioned. Among these, coenzyme Q10 is preferable.

The proportion of the ingredient(s) (B) added to the composition of the invention is not limited and can be suitably adjusted according to the form of the composition, the expected effect, and the like. As one example, the total proportion of the ingredient(s) (B) is in the range of 0.00001 to 100 parts by weight per part by weight of the total weight of ingredient(s) (A). More specifically, when the ingredient(s) (B) is arbutin, ellagic acid, 4-alkylresorcinol, tranexamic acid, and/or salts thereof, the total proportion of the ingredient(s) (B) is preferably 0.1 to 100 parts by weight, and more preferably 1 to 100 parts by weight, per part by weight of the total weight of ingredient(s) (A). For example, when the ingredient(s) (B) is chamomile extract, the total proportion of the ingredient(s) (B) is preferably 0.00001 to 10 parts by weight, and more preferably 0.0001 to 10 parts by weight, per part by weight of the total weight of ingredient (s) (A). As another example, when the ingredient(s) (B) is ubiquinone, the total proportion of the ingredient(s) (B) is preferably 0.001 to 100 parts by weight, and more preferably 0.01 to 100 parts by weight, per part by weight of a total weight of ingredient(s) (A). In the above-mentioned proportions of the ingredient(s) (B), the proportion of chamomile extract is calculated in terms of solid content. With the combined use of the ingredients (A) and (B) in the above-mentioned proportions, the effects of preventing or improving skin pigmentation of both the ingredients (A) and (B) are synergistically enhanced, whereby more excellent pigmentation inhibitory effects are exhibited.

The total proportion of ingredient(s) (B) is, for example, 0.0001 to 50% by weight based on the total weight of the composition for preventing or improving skin pigmentation. More specifically, when the ingredient(s) (B) is arbutin, ellagic acid, 4-alkylresorcinol, tranexamic acid, and/or salts thereof, the total proportion of the ingredient(s) (B) is, for example, 0.01 to 50% by weight, preferably 0.05 to 30% by weight, and more preferably 0.1 to 20% by weight. When the ingredient(s) (B) is chamomile extract, the total proportion of the ingredient(s) (B) is, for example, 0.0001 to 0.5% by weight, preferably 0.0005 to 0.3% by weight, and more preferably 0.001 to 0.2% by weight. When the ingredient (s) (B) is ubiquinone, the total proportion of the ingredient(s) (B) is, for example, 0.0001 to 50% by weight, preferably 0.001 to 30% by weight, and more preferably 0.01 to 20% by weight. In the above-mentioned proportions of the ingredient(s) (B), the proportion of chamomile extract is calculated in terms of solid content.

The composition for preventing or improving skin pigmentation of the invention is formed into cosmetic compositions, externally-applied pharmaceutical compositions, and the like so that it is applied through the skin. In the invention, the externally-applied pharmaceutical compositions encompass externally-applied medical or quasi-medical drugs. Such externally-applied pharmaceutical compositions for preventing or improving skin pigmentation can take any form without limitation insofar as they are applicable to the skin or mucosa, such as an aqueous solution, a solubilized form, an emulsified form, a dispersed powder, a water/oil two-layer type, etc. Specific examples include solutions, oily preparations, lotions, liniments, emulsions, suspensions, creams, ointments, etc. Examples of cosmetic compositions include lotions; emollient emulsions, milky lotions, nourishing emulsions, cleansing emulsions, and like emulsions; emollient creams, massage creams, cleansing creams, makeup creams, and like creams; etc. The composition for preventing or improving pigmentation of the invention may also be formed into hair care products such as hair growth formula and the like. Examples of hair care products include hair tonics, hair creams, hair lotions, aerosols (air sprays), mousses, shampoos, rinses, liquids, etc.

The composition for preventing or improving skin pigmentation of the invention may contain a wide range of known components added to compositions applied to the skin or mucosa, such as cosmetic compositions, externally-applied pharmaceutical compositions, and the like. Examples of such ingredients include humectants, UV absorbers, UV dispersants, vitamins, plant extracts, astringents, anti-inflammatory agents (antiphlogistic agents), whiteners, cell activators, vasodilators, blood circulation accelerators, skin function accelerators, and the like in addition to surfactants, coloring agents (dyes, pigments), perfumes, preservatives, bactericides (antibacterials), thickeners, antioxidants, sequestering agents, refrigerants, deodorizers, and the like. Known bases or carriers can also be used in accordance with the above-mentioned various forms.

Among the above-mentioned ingredients, examples of surfactants include anionic surfactants, such as salts of higher fatty acids, alkylsulfonates, polyoxyethylene alkyl ether sulfates, alkyl ether phosphates, *N*-acylamino acid salts, acyl *N*-methyltaurates, etc.; cationic surfactants, such as alkyltrimethyl ammonium chlorides, dialkyldimethyl ammonium chlorides, etc.; amphoteric surfactants, such as alkyl dimethylaminoacetic acid betaines, alkylamidodimethylaminoacetate betaines, 2-alkyl-*N*-carboxy-*N-*hydroxyimidazolinium betaines, etc.; nonionic surfactants, such as polyoxyethylene types, polyhydric alcohol ester types, ethyleneoxide propyleneoxide block polymers, etc. Any high molecular weight surfactants or natural surfactants can also be used without limitation.

Examples of preservatives include ethyl *p-*hydroxybenzoate, salicylic acid, sorbic acid, etc. Examples of thickeners include xanthane gum, carboxymethyl cellulose sodium, carboxy vinyl polymers, etc. Examples of sequestering agents include sodium salts of ethylenediamine tetra-acetic acid, phosphoric acid, citric acid, etc.

The composition for preventing or improving skin pigmentation can be directly applied to or sprayed onto the skin in accordance with the form. The amount in a single dose and the number of doses per day of the composition for preventing or improving skin pigmentation are determined according to the age of the user (human), the gender, the intended use, the condition of the affected part of the skin, etc. For example, a suitable amount of the composition can be applied to the skin from once to 5 or 6 times per day.

The composition for preventing or improving skin pigmentation can prevent or improve pigmentation of the skin, and therefore is useful as a skin-lightening composition, skin anti-aging composition, skin-dullness improving composition, or melasma improving composition.

### 2. Non-Therapeutic Method for preventing or improving pigmentation

The invention also provides a non-therapeutic method for preventing or improving pigmentation of the skin. More specifically, the method comprises applying, to the human skin, at least one member (A) selected from the group consisting of adenosine 5'-monophosphates and salts thereof, and at least one member (B) selected from the group consisting of arbutins, ellagic acid, 4-alkylresorcinols, tranexamic acid, salts thereof, chamomile extracts, and ubiquinones.

In the method of the invention, the kinds of ingredient(s) (A), the kinds of ingredients (B), and the ratio of the ingredients (A) to (B) are as mentioned above. The manner of applying the ingredients (A) and (B) to the skin and the number of times the ingredients (A) and (B) are applied per day are also as mentioned above.

The amounts of the ingredients (A) and (B) applied to the skin may be adjusted to reach the effective amounts for enhancing the action of preventing or improving skin pigmentation by the combined used of the ingredients (A) and (B).

The method is suitably carried out by applying, to the skin, the effective amount of the above-mentioned composition for preventing or improving skin pigmentation.

The method is useful as a cosmetic method. The method can effectively prevent or improve pigmentation of the skin, and therefore is useful as skin-lightening methods, skin anti-aging methods, skin-dullness improving methods, or melasma improving methods.

### EXAMPLES

The present invention is described with reference to Examples and Test Examples, but is not limited thereto. In the following Examples and Test Examples, "chamomile liquid" (liquid, 0.9% by weight of solid content, manufactured by ICHIMARU PHARCOS CO.,LTD) was used as chamomile extract. The amount of chamomile extract refers to the amount of "chamomile liquid".

### Example 1 Lotion

| | (% by weight) |
|---|---|
| AMP | 2.0 |
| Arbutin | 3.0 |
| 1, 3-butylene glycol | 2.0 |
| Concentrated glycerin | 2.0 |
| Polyoxyethylenemethyl polysiloxane copolymer | 0.3 |
| Ethanol | 5.0 |
| Preservative | Suitable amount |
| pH adjuster | Adjusted to pH 6.5 |
| Purified water | Remainder |
| Total | 100.0 |

### Example 2 Lotion

| | (% by weight) |
|---|---|
| AMP | 2.0 |
| Tranexamic acid | 3.0 |
| Dipropylene glycol | 3.0 |
| Concentrated glycerin | 2.0 |
| Polyoxyethylene sorbitan monostearate (20E.0.) | 1.0 |
| Ethanol | 10.0 |
| Preservative | Suitable amount |
| pH adjuster | Adjusted to pH 6.5 |
| Perfume | Suitable amount |
| Purified water | Remainder |
| Total | 100.0 |

### Example 3 Cream

| | (% by weight) |
|---|---|
| AMP | 2.0 |
| Ellagic acid | 0.5 |
| Polyoxyethylene alkylene alkyl-comodified silicone | 2.0 |
| Decamethylcyclopentasiloxane | 18.0 |
| Liquid paraffin | 4.0 |
| Concentrated glycerin | 3.0 |
| 1,3-butylene glycol | 3.0 |
| Ethanol | 5.0 |
| Preservative | Suitable amount |
| pH adjuster | Adjusted to pH 6.5 |
| Purified water | Remainder |
| Total | 100.0 |

### Example 4 Treatment lotion

| | (% by weight) |
|---|---|
| AMP2Na | 1.5 |
| Chamomile extract | 5.0 |
| (trade name: "chamomile liquid", manufactured by ICHIMARU PHARCOS CO.,LTD) | |
| Dipropylene glycol | 3.0 |
| Concentrated glycerin | 3.0 |
| Sodium hyaluronate | 0.2 |
| Xanthan gum | 0.2 |
| Polyoxyethylenemethyl polysiloxane copolymer | 0.3 |
| Ethanol | 3.0 |
| Preservative | Suitable amount |
| pH adjuster | Adjusted to pH 6.5 |
| Perfume | Suitable amount |
| Purified water | Remainder |
| Total | 100.0 |

### Example 5 Milky lotion

| | (% by weight) |
|---|---|
| AMP2Na | 1.0 |
| 4-hexylresorcinol | 0.5 |
| Ascorbyl tetra 2-hexyldecanate | 2.0 |
| Polyoxyalkylene alkyl-comodified silicone | 1.0 |
| Decaglycerol monostearate | 2.0 |
| Glycerol monostearate | 1.0 |
| Stearic acid | 3.0 |
| Behenyl alcohol | 2.0 |
| Tri-2-ethyl hexane acid glycerol | 5.0 |
| Squalan | 2.0 |
| Decamethylcyclopentasiloxane | 1.0 |
| Hydrogenated soybean phosphatide | 0.3 |
| dl-α-tocopherol acetate | 0.1 |
| Concentrated glycerin | 3.0 |
| 1, 3-butylene glycol | 3.0 |
| Carboxyvinyl polymer | 0.3 |
| Preservative | Suitable amount |
| pH adjuster | Adjusted to pH 6.5 |
| Purified water | Remainder |
| Total | 100.0 |

### Example 6 Lotion (not part of the invention)

| | (% by weight) |
|---|---|
| AMP2Na | 1.5 |
| Linolic acid | 0.1 |
| Hydrogenated soybean phosphatide | 1.0 |
| Dipropylene glycol | 5.0 |
| Ethanol | 3.0 |
| Preservative | Suitable amount |
| pH adjuster | Adjusted to pH 6.5 |
| Purified water | Remainder |
| Total | 100.0 |

### Example 7 Cream (not part of the invention)

| | (% by weight) |
|---|---|
| AMP2Na | 1.5 |
| Linolic acid | 2.0 |
| Stearic acid | 3.0 |
| Polyethylene glycol monostearate (40E.O.) | 2.0 |
| Self-emulsifying glycerol monostearate | 5.0 |
| Tri-2-ethyl-hexanoic-acid glycerol | 15.0 |
| Behenyl alcohol | 1.0 |
| Squalane | 10.0 |
| Concentrated glycerin | 5.0 |
| Preservative | Suitable amount |
| pH adjuster | Adjusted to pH 6.5 |
| Perfume | Suitable amount |
| Purified water | Remainder |
| Total | 100.0 |

### Test Example 1: Evaluation of the pigmentation improvement effect-1

The following tests were performed using colored guinea pigs for evaluating the pigmentation improving effect obtained by the combined use of AMP and arbutin.

### <Preparation of a sample and production of an animal model>

### 1. Preparation of a test solution

Using a 20% by weight ethanol aqueous solution as a base material, a mixed solution of 3% by weight AMP2Na and 3% by weight arbutin (β type) (Formulation Example 1), a solution comprising a 3% by weight AMP2Na (Comparative Formulation Example 1-1), and a solution comprising 3% by weight arbutin (β type) (Comparative Formulation Example 1-2) were prepared.

### 2. Production of an animal model with skin pigmentation

The hair on the back of eight colored guinea pigs was shaved, and the shaved back of each guinea pig was exposed to ultraviolet irradiation several times, thereby producing four skin pigmentation sites per animal.

### <Test Procedure>

11 days after the final ultraviolet irradiation exposure for the guinea pigs, one of each test solution was applied twice per day for 14 days. Each of the four pigmentation sites of each colored guinea pig was applied with a suitable amount of one solution, from among the three test solutions (Formulation Example 1, and Comparative Formulation Examples 1-1 and 1-2) and a 20% by weight ethanol aqueous solution (control). Fourteen days later, 5 judgment persons compared the skin brightness between each pigmentation site, to which one solution, from among the three test solutions was applied and the site to which the control was applied, according to t Scheffe's paired comparison method, and graded the comparison results in accordance with the judgment criteria shown in Table 1. Table 1

| Judgment criteria | |
|---|---|
| The brightness of the pigmentation site to which the test solution was applied was much higher than that of the pigmentation site to which the control was applied. | 3 |
| The brightness of the pigmentation site to which the test solution was applied was noticeably higher than that of the pigmentation site to which the control was applied. | 2 |
| The brightness of the pigmentation site to which the test solution was applied was slightly higher compared with the pigmentation site to which the control was applied. | 1 |
| The brightness of the pigmentation site to which the test solution was applied was the same as that of the pigmentation site to which the control was applied. | 0 |
| The brightness of the pigmentation site to which the test solution was applied was slightly lower than that of the pigmentation site to which the control was applied. | -1 |
| The brightness of the pigmentation site to which the test solution was applied was noticeably lower than that of the pigmentation site to which the control was applied. | -2 |
| The brightness of the pigmentation site to which the test solution was applied was much lower than that of the pigmentation site to which the control was applied. | -3 |

### <Calculation method>

Before the application and 14 days after the application, 5 judgment persons compared the three pigmentation sites of the guinea pigs, each of which was applied with one solution, from among the three test solutions, with the pigmentation site to which the control was applied and graded the comparison. The total of the visual grading scores for each pigmentation site obtained from each of the five judgment persons was defined as the visual grading scores for each test solution. The average visual grading scores for each test solution was calculated from eight colored guinea pigs. The delta value (Δ) of visual grading score was calculated by subtracting the visual grading scores before the application of the test solution from the visual grading scores 14 days after the application. A higher calculated delta value (Δ) of visual grading score shows that the skin color of the pigmentation site to which the test solution was applied was lighter than that of the pigmentation site to which the base agent was applied, and a higher delta value (A) of visual grading score shows that the pigmentation improvement effect is high.

### <Results and Considerations>

The obtained results are shown in Figure 1, in which the delta values (Δ) of visual grading scores that were obtained by subtracting the visual grading scores before the application of the test solution from the visual grading scores 14 days after the application. The delta values (Δ) of visual grading scores of Comparative Formulation Examples 1-1 and 1-2 were 1.00 and 0.50, respectively, and in contrast, the delta value (Δ) of visual grading score of the solution of Formulation Example 1 was 5.50. The fact that the sum of the delta values (Δ) of visual grading scores of Comparative Formulation Examples 1-1 and 1-2 was 1.50 and the delta value (A) of visual grading score of Formulation Example 1 was 5.50 clearly showed that the pigmentation improvement effect is synergistically enhanced by the combined use of AMP2Na and arbutin in Formulation Example 1.

### Test Example 2: Evaluation of the pigmentation improvement effect-2

### <Preparation of a sample and production of an animal model>

### 1. Preparation of a test solution

Using a 20% by weight ethanol aqueous solution as a base material, a mixed solution of 3% by weight AMP2Na and 0.5% by weight ellagic acid hydrate (Formulation Example 2), a solution comprising 3% by weight AMP2Na (Comparative Formulation Example 2-1), and a solution comprising 0.5% by weight ellagic acid hydrate (Comparative Formulation Example 2-2) were prepared.

### 2. Production of an animal model with pigmentation

The hair on the back of eight colored guinea pigs was shaved, and the shaved back of each guinea pig was exposed to ultraviolet irradiation several times, thereby producing four skin pigmentation sites per animal.

### <Test Procedure>

The experiment was performed using the eight colored guinea pigs, following the procedure of Test Example 1, except that the test solution was applied twice per day for 28 days.

### <Calculation Method>

The delta value (Δ) of visual grading score was calculated in the same manner as in Test Example 1 above.

### <Results and Considerations>

The obtained results are shown in Figure 2, in which the delta values (Δ) of visual grading scores that were obtained by subtracting the visual comparison grade before the application of the test solution from the visual grading scores 28 days after the application. The delta values (Δ) of visual grading scores of Comparative Formulation Examples 2-1 and 2-2 were 1.25 and 3.25, respectively, and in contrast, the delta value (Δ) of visual grading score of the solution of Formulation Example 2 was 10.50. The fact that the sum of the delta values (Δ) of visual grading scores of Comparative Formulation Examples 2-1 and 2-2 was 4.50 and the delta value (Δ) of visual grading score of Formulation Example 2 was 10.50 clearly showed that the pigmentation improvement effect is synergistically enhanced by the combined use of AMP2Na and ellagic acid hydrate in Formulation Example 2.

### Test Example 3: Evaluation of the pigmentation improvement effect-3

The following test was performed using colored guinea pigs for evaluating the effect of improving pigmentation obtained by the combined use of AMP and tranexamic acid.

### <Preparation of a sample and production of an animal model>

### 1. Preparation of a test solution

Using a 20% by weight ethanol aqueous solution as a base material, a mixed solution of 3% by weight AMP2Na and 3% by weight tranexamic acid (Formulation Example 3), a solution comprising 3% by weight AMP2Na (Comparative Formulation Example 3-1), and a solution comprising 3% by weight tranexamic acid (Comparative Formulation Example 2-2) were prepared.

### 2. Production of an animal model with pigmentation

The hair on the back of eight colored guinea pigs was shaved, and the shaved back of each guinea pig was exposed to ultraviolet irradiation several times, thereby producing four skin pigmentation sites per animal.

### <Test Procedure>

Following the procedure of Test Example 1, the test was performed using the eight colored guinea pigs.

### <Calculation Method>

The delta value (Δ) of visual grading score was calculated in the same manner as in Test Example 1 above.

### <Results and Considerations>

The obtained results are shown in Figure 3, in which the delta values (Δ) of visual grading scores that were obtained by subtracting the visual grading scores before the application of the test solution from the visual grading scores 14 days after the application. The delta values (Δ) of visual grading scores of Comparative Formulation Examples 3-1 and 3-2 were 1.75 and 1.25, respectively, and in contrast, the delta value (Δ) of visual grading score of the solution of Formulation Example 3 was 4.50. The fact that the sum of the delta values (Δ) of visual grading scores of Comparative Formulation Examples 3-1 and 3-2 was 3.00 and the delta value (Δ) of visual grading scores of Formulation Example 3 was 4.50 clearly showed that the pigmentation improvement effect is synergistically enhanced by the combined use of AMP2Na and tranexamic acid in Formulation Example 3.

### Test Example 4: Evaluation of the pigmentation improvement effect-4

### <Preparation of a sample and production of an animal model>

### 1. Preparation of a test solution

Using a 20% by weight ethanol aqueous solution as a base material, a mixed solution of 3% by weight AMP2Na and 0.3% by weight 4-n-hexylresorcinol (Formulation Example 4), a solution comprising 3% by weight AMP2Na (Comparative Formulation Example 4-1), and a solution comprising 0.3% by weight 4-n-hexylresorcinol (Comparative Formulation Example 4-2) were prepared.

### 2. Production of an animal model with pigmentation

The hair on the back of eight colored guinea pigs was shaved, and the shaved back of each guinea pig was exposed to ultraviolet irradiation several times, thereby producing four skin pigmentation sites per animal.

### <Test Procedure and Calculation Method>

Following the procedure of Comparative Test Example 1, the test was performed and the delta value (Δ) of visual grading score was calculated.

### <Results and Considerations>

The obtained results are shown in Figure 4, in which the delta values (Δ) of visual grading scores that were obtained by subtracting the visual grading scores before the application of the test solution from the visual comparison grade 14 days after the application. The delta values (Δ) of visual grading scores of Comparative Formulation Examples 4-1 and 4-2 were 1.25 and 1.75, respectively, and in contrast, the delta value (Δ) of visual grading score of the solution of Formulation Example 4 was 7.25. The fact that the sum of the delta values (Δ) of visual grading scores of Comparative Formulation Examples 4-1 and 4-2 was 3.00 and the delta value (Δ) of visual grading score of Formulation Example 4 was 7.25 clearly showed that the pigmentation improvement effect is synergistically enhanced by the combined use of AMP2Na and 4-n-hexylresorcinol in Formulation Example 4.

### Test Example 5: Evaluation of the pigmentation improvement effect-5

### <Preparation of a sample and Production of an animal model>

### 1. Preparation of a test solution

Using a 20% by weight ethanol aqueous solution as a base material, a mixed solution of 3% by weight AMP2Na and 5% by weight chamomile extract (trade name "chamomile liquid", manufactured by ICHIMARU PHARCOS CO., LTD) (Formulation Example 5), a solution comprising 3% by weight AMP2Na (Comparative Formulation Example 5-1), and a solution comprising 5% by weight chamomile extract (Comparative Formulation Example 5-2) were prepared.

### 2. Production of an animal model with pigmentation

The hair on the back of eight colored guinea pigs was shaved, and the shaved back of each guinea pig was exposed to ultraviolet irradiation several times, thereby producing four skin pigmentation sites per animal.

### <Test Procedure and Calculation Method>

Following the procedure of Comparative Test Example 1, the test was performed and the delta value (Δ) of visual grading score was calculated.

### <Results and Considerations>

The obtained results are shown in Figure 5, in which the delta values (Δ) of visual grading scores that were obtained by subtracting the visual grading scores before the application of the test solution from the visual grading scores 14 days after the application. The delta values (Δ) of visual grading scores of Comparative Formulation Examples 5-1 and 5-2 were 1.13 and 0.50, respectively, and in contrast, the delta value (Δ) of visual grading score of the solution of Formulation Example 5 was 3.00. The fact that the sum of the delta values (Δ) of visual grading scores of Comparative Formulation Examples 5-1 and 5-2 was 1.63 and the delta value (Δ) of visual grading score of Formulation Example 5 was 3.00 clearly showed that the pigmentation improvement effect is synergistically enhanced by the combined use of AMP2Na and 4-n-hexylresorcinol.

### Test Example 6: Evaluation of the pigmentation improvement effect-6

### <Preparation of a sample and production of an animal model>

### 1. Preparation of a test solution

Using a 20% by weight ethanol aqueous solution as a base material, a mixed solution of 3% by weight AMP2Na and 1% by weight coenzyme Q10 (Formulation Example 6), a solution comprising 3% by weight AMP2Na (Comparative Formulation Example 6-1), and a solution comprising 1% by weight coenzyme Q10 (Comparative Formulation Example 6-2) were prepared.

### 2. Production of an animal model with pigmentation

The hair on the back of eight colored guinea pigs was shaved, and the shaved back of each guinea pig was exposed to ultraviolet irradiation several times, thereby producing four skin pigmentation sites per animal.

### <Test Procedure and Calculation Method>

Following the procedure of Comparative Test Example 1, the test was performed and the delta value (Δ) of visual grading score was calculated.

### <Results and Considerations>

The obtained results are shown in Figure 6, in which the delta values (A) of visual grading scores that were obtained by subtracting the visual comparison grade before the application of the test solution from the visual grading scores 14 days after the application. The delta values (A) of visual grading scores of Comparative Formulation Examples 6-1 and 6-2 were 1.25 and 0.63, respectively, and in contrast, the delta value (A) of visual grading score of the solution of Formulation Example 6 was 3.88. The fact that the sum of the delta values Δ) of visual grading scores of Comparative Formulation Examples 6-1 and 6-2 was 1.88 and the delta value (Δ) of visual grading score of Formulation Example 6 was 3.88 clearly showed that the pigmentation improvement effect is synergistically enhanced by the combined use of AMP2Na coenzyme Q10.

### INDUSTRIAL APPLICABILITY

The composition for preventing or improving skin pigmentation of the invention comprises AMP or a salt thereof as an ingredient (A) and a specific melanin formation inhibitor or ubiquinone as an ingredient (B) in combination, and therefore the actions of preventing or improving skin pigmentation of both ingredients (A) and (B) are synergistically enhanced. Thus, since the composition for preventing or improving skin pigmentation of the invention exhibits a superior skin pigmentation prevention or improvement effect, the composition of the invention is useful as a cosmetics and externally-applied preparation for the skin (pharmaceutical composition) for the purpose of skin-lightening, skin anti-aging, reduction of skin dullness, and amelioration of melasma.

The ingredients (A) and (B) used as an active ingredient in the composition for preventing or improving skin pigmentation of the invention are confirmed to be highly safe, and thus can be used daily as a cosmetic material. Therefore, users can prevent or improve skin pigmentation simply and easily by the daily use, as cosmetics, of the composition for preventing or improving pigmentation of the invention.

## Claims

1. A cosmetic composition comprising:
at least one member (A) selected from the group consisting of adenosine 5'-monophosphates and salts thereof; and
at least one member (B) selected from the group consisting of arbutins, ellagic acid, 4-alkylresorcinols, tranexamic acid, salts thereof, chamomile extracts, and ubiquinones.

2. A composition according to claim 1, comprising said at least one member (A) in a total proportion of 0.05 to 10% by weight based on a total weight of the composition.

3. A composition according to claim 1, comprising said at least one member (B) in a total proportion of 0.00001 to 100 parts by weight per part by weight of a total weight of said at least one member (A).

4. A composition according to claim 1, comprising said at least one member (B) in a total proportion of 0.0001 to 50% by weight based on a total weight of the composition.

5. A non-therapeutic method for preventing pigmentation or for skin lightening, reduction of skin dullness or amelioration of melasma, comprising applying, to human skin, at least one member (A) selected from the group consisting of adenosine 5'-monophosphates and salts thereof and at least one member (B) selected from the group consisting of arbutins, ellagic acid, 4-alkylresorcinols, tranexamic acid, salts thereof, chamomile extracts, and ubiquinones.

## Patentansprüche

1. Kosmetikzusammensetzung, umfassend:
mindestens ein Element (A), ausgewählt aus der Gruppe bestehend aus Adenosin-5'-monophosphaten und Salzen davon; und
mindestens ein Element (B), ausgewählt aus der Gruppe bestehend aus Arbutinen, Ellagsäure, 4-Alkylresorcinolen, Tranexamsäure, Salzen davon, Kamillenextrakten und Ubichinonen.

2. Zusammensetzung gemäss Anspruch 1, umfassend das mindestens eine Element (A) in einem Gesamtanteil von 0,05 bis 10 Gew.% auf Basis des Gesamtgewichts der Zusammensetzung.

3. Zusammensetzung gemäss Anspruch 1, umfassend das mindestens eine Element (B) in einem Gesamtanteil von 0,00001 bis 100 Gew.-Teilen pro Gew.-Teil des Gesamtgewichts des mindestens einen Elements (A).

4. Zusammensetzung gemäss Anspruch 1, umfassend das mindestens eine Element (B) in einem Gesamtanteil von 0,0001 bis 50 Gew.% auf Basis des Gesamtgewichts der Zusammensetzung.

5. Nicht-therapeutisches Verfahren zur Prävention von Pigmentierung oder zur Hautaufhellung, Reduzierung der Hautstumpfheit oder Linderung von Melasma, umfassend das Auftragen mindestens eines Elements (A), ausgewählt aus der Gruppe bestehend aus Adenosin-5'-monophosphaten und Salzen davon, und mindestens eines Elements (B), ausgewählt aus der Gruppe bestehend aus Arbutinen, Ellagsäure, 4-Alkylresorcinolen, Tranexamsäure, Salzen davon, Kamillenextrakten und Ubichinonen, auf menschliche Haut.

## Revendications

1. Composition cosmétique comprenant :
au moins un élément (A) choisi dans le groupe constitué par les adénosines 5'-monophosphates et leurs sels ; et
au moins un élément (B) choisi dans le groupe constitué par les arbutines, l'acide ellagique, les 4-alkylrésorcinols, l'acide tranéxamique, leurs sels, des extraits de camomille, et les ubiquinones.

2. Composition selon la revendication 1, comprenant ledit au moins un élément (A) dans une proportion totale de 0,05 à 10 % en poids basée sur un poids total de la composition.

3. Composition selon la revendication 1, comprenant ledit au moins un élément (B) dans une proportion totale de 0,00001 à 100 parties en poids par partie en poids d'un poids total dudit au moins un élément (A).

4. Composition selon la revendication 1, comprenant ledit au moins un élément (B) dans une proportion totale de 0,0001 à 50 % en poids basée sur un poids total de la composition.

5. Procédé non thérapeutique de prévention de pigmentation ou d'éclaircissement de la peau, de réduction du manque d'éclat de la peau ou d'amélioration de mélasme, comprenant l'application, à une peau humaine, d'au moins un élément (A) choisi dans le groupe constitué par les adénosines 5'-monophosphates et leurs sels et d'au moins un élément (B) choisi dans le groupe constitué par les arbutines, l'acide ellagique, les 4-alkylrésorcinols, l'acide tranéxamique, leurs sels, des extraits de camomille, et les ubiquinones.
